# FASCICULE DE BREVET EUROPEEN

(11) **EP 0 850 635 B1**
(45) Date de publication et mention de la délivrance du brevet: **14.07.1999**
(21) Numéro de dépôt: 97402833.4
(22) Date de dépôt: 25.11.1997
(51) Int. Cl.: A61K 7/13

(54) **Procédé de teinture d'oxydation en deux temps des fibres kératiniques avec un sel ou un complexe de manganèse et un 1-naphtol 4-substitué et kit de teinture**
Oxidationsfärbungsverfahren in zwei Stufen von keratinischen Fasern mit einem Mangansalz oder einem Komplex von Mangan und ein 1-Naphtol, substituiert in Lage 4 und eine Färbeset
Process for oxydative dyeing of keratinic fibers in two steps with a manganese salt or a manganese complex and a 1-naphtol substituted in position 4 and a dyeing set

(30) Priorité: 23.12.1996 FR 9615893
(43) Date de publication de la demande: 01.07.1998
(73) Titulaire: L'OREAL, 75008 Paris (FR)
(72) Inventeur: Henrion, Jean-Christophe, 93500 Pantin (FR); Andrean, Hervé, 75014 Paris (FR)
(74) Mandataire: Goulard, Sophie

(56) Documents cités:
- EP-A- 0 375 977
- DE-A- 4 234 887
- FR-A- 2 090 272
- GB-A- 2 187 210

## Description

L'invention a pour objet un nouveau procédé de teinture d'oxydation en deux temps des fibres kératiniques et en particulier des fibres kératiniques humaines telles que les cheveux mettant en oeuvre, dans un premier temps, un sel de manganèse et/ou un complexe de manganèse, et dans un deuxième temps, un 1-naphtol 4-substitué en milieu oxydant présentant un pH supérieur ou égal à 6.

L'invention a également pour objet le dispositif à plusieurs compartiments, ou "kit de teinture", destiné à la mise en oeuvre du procédé de teinture selon l'invention.

Il a déjà été proposé, notamment dans la demande de brevet FR-A-758 685, de teindre les fibres animales telles que la peau, les poils, les plumes et le cuir, à l'aide de colorants de type 4-alcoxy 1-naphtol, cependant les conditions de teinture qui sont décrites dans ce document ne sont pas applicables à la teinture des cheveux humains. En effet, selon ce document, la teinture est de préférence réalisée par imprégnation de la matière à teindre pendant plusieurs heures dans une solution de soude contenant un 4-alcoxy 1-naphtol en présence d'un agent oxydant. Ces conditions drastiques ne sont pas acceptables en cosmétique.

Le document FR-A-2 090 272 décrit la teinture des cheveux et l'enlèvement de teintures de cheveux ayant subi une coloration.

D'autre part, il est bien connu que l'oxydation des naphtols conduit à la formation de pigments de la famille des indigoïdes aux nuances variées qui sont toujours recherchées dans le domaine de la coloration des fibres kératiniques humaines telles que les cheveux.

La coloration des fibres kératiniques humaines doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Or, la demanderesse vient maintenant de découvrir qu'il est possible d'obtenir avec des composés de type 1-naphtol 4-substitués, de façon rapide, des colorations particulièrement puissantes et résistantes, en appliquant, de façon séparée et étalée dans le temps (deux temps), d'abord une composition A contenant au moins un sel de manganèse et/ou au moins un complexe de manganèse, puis une composition B présentant un pH supérieur ou égal à 6 et résultant du mélange extemporané (juste avant emploi) d'une composition B1 contenant au moins un composé de type 1-naphtol 4-substitué de formule (I) telle que définie ci-après et d'une composition B2 contenant au moins un agent oxydant.

Cette découverte est la base de la présente invention.

L'invention a donc pour objet un procédé de teinture d'oxydation en deux temps des fibres kératiniques et en particulier des fibres kératiniques humaines telles que les cheveux, caractérisé par le fait qu'on applique sur ces fibres :
- dans un premier temps, au moins une composition A contenant, dans un milieu approprié pour la teinture, au moins un sel de manganèse et/ou au moins un complexe de manganèse,
- et dans un deuxième temps, au moins une composition B présentant un pH supérieur ou égal à 6 et résultant du mélange extemporané :
   a) d'une composition B1 contenant, dans un milieu approprié pour la teinture, au moins un 1-naphtol 4-substitué de formule (I) suivante : 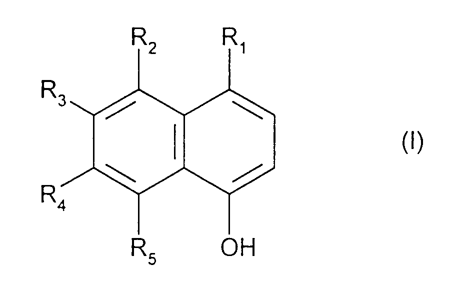 dans laquelle :
      R₁ représente un atome d'halogène tel que le brome, le chlore, l'iode ou le fluor ; un radical benzyle ; un radical benzyloxy ; un radical alcoxy linéaire, ramifié ou cyclique en C₁-C₂₉ pouvant être substitué par un ou plusieurs atomes d'halogène et/ou interrompu par un ou plusieurs hétéroatomes ; un radical acyloxy en C₁-C₂₉ ou un radical alcoxycarboxylique en en C₁-C₂₉ ;
      R₂, R₃, R₄ et R₅, identiques ou différents, représentent un atome d'hydrogène, un atome d'halogène tel que le brome, le chlore, l'iode ou le fluor, un radical hydroxyle, alkyle linéaire ou ramifié en C₁-C₂₉, alcoxy linéaire ou ramifié en C₁-C₂₉, acyle ou benzyloxy,
   b) et d'une composition B2 contenant, dans un milieu approprié pour la teinture, au moins un agent oxydant.

Dans la formule (I) ci-dessus, les radicaux alkyle, alcoxy et acyle ont de préférence de 1 à 12 atomes de carbone et encore plus préférentiellement de 1 à 6 atomes de carbone.

Les colorations obtenues selon le procédé de l'invention sont puissantes et présentent une excellente ténacité vis à vis des différents traitements que peuvent subir les fibres kératiniques. De plus, le procédé de teinture d'oxydation conforme à l'invention permet de teindre les fibres kératiniques de façon rapide, dans des conditions tout à fait adaptées aux exigences cosmétiques, c'est à dire des conditions ménageant l'intégrité des fibres kératiniques.

Selon le procédé de l'invention, l'application de la composition A sur les fibres peut être séparée de l'application de la composition B sur ces mêmes fibres par une étape intermédiaire d'essorage et/ou de rinçage.

Selon une forme de réalisation particulière et préférée du procédé selon l'invention, la composition A est appliquée sur les fibres kératiniques avec un temps de pose compris de préférence entre 5 secondes et 10 minutes environ et encore plus préférentiellement entre 30 secondes et 5 minutes environ, puis la composition B est appliquée sur ces fibres avec un temps de pose compris de préférence entre 30 secondes et 45 minutes environ et encore plus préférentiellement entre 3 et 30 minutes environ.

Le ou les sels de manganèse pouvant être utilisés dans la composition A selon le procédé de teinture de l'invention n'ont pas d'activité oxydante propre et dans ces sels, le manganèse présente de préférence un degré d'oxydation égal à 2 ou à 3. On peut bien entendu utiliser un ou plusieurs sels de manganèse.

Ces sels de manganèse sont de préférence choisis parmi les sels hydrosolubles, minéraux ou organiques du manganèse tels que le dichlorure de manganèse, le trichlorure de manganèse, les sulfates de manganèse, les nitrates de manganèse, le diacétate de manganèse, les carbonates de manganèse, les dihydrogénocarbonates de manganèse, l'acétylacétonate de manganèse, le triacétate de manganèse, le lactate de manganèse, le formate de manganèse, l'acétyl méthionate de manganèse, le gluconate de manganèse, et leurs hydrates.

Le ou les complexes de manganèse pouvant être utilisés dans la composition A selon le procédé de teinture de l'invention n'ont pas d'activité oxydante propre et sont de préférence choisis parmi les complexes de manganèse dans lesquels le manganèse présente un degré d'oxydation égal à 4, tels que ceux décrits par exemple dans le brevet US 5, 194, 416. Parmi ces complexes de manganèse, on peut plus particulièrement citer le bis-(hexafluorophosphate) de bis-(octahydro-1, 4 ,7-triméthyl-1H-1, 4, 7-triazonine-N1, N4, N7) tri-µ-oxo di-manganèse (R.N. 116633-52-4).

Le diacétate de manganèse tétrahydrate et le bis-(hexafluorophosphate) de bis-(octahydro-1, 4 ,7-triméthyl-1H-1, 4, 7-triazonine-N1, N4, N7) tri-µ-oxo di-manganèse sont particulièrement préférés.

Selon l'invention, le ou les sels de manganèse et/ou le ou les complexes de manganèse sont de préférence présents à une concentration comprise entre 0,0001 et 1 % en poids environ d'équivalents métal par rapport au poids total de la composition A. Encore plus préférentiellement, cette concentration est comprise entre 0,001 et 0,15 % en poids environ d'équivalents métal par rapport au poids total de la composition A.

Parmi les composés de formule (I) conformes à l'invention, on peut plus particulièrement citer :
- le 4-benzyl-naphtalèn-1-ol,
- le 4-chloro naphtalèn-1-ol,
- le 4-chloro-5,8-diméthoxy-6-méthyl- naphtalèn-1-ol,
- le 4-chloro-5,8-diméthoxy- naphtalèn-1-ol,
- le 4-acétoxy-5-chloro-6-méthyl-7-acétyl-8-hydroxy-naphtalèn-1-ol,
- le 4-acétoxy-6-méthyl-7-acétyl-8-hydroxy-naphtalèn-1-ol,
- le 4-acétoxy-8-benzyloxy-naphtalèn-1-ol,
- le 4-benzyloxy-naphtalèn-1-ol,
- le 4,8-dibenzyloxy-6-méthyl-naphtalèn-1-ol,
- le 4,8-dibenzyloxy-naphtalèn-1-ol,
- le 4-benzyloxy-8-(2-chloro)éthoxy-naphtalèn-1-ol,
- le 4-benzyloxy-8-isopropyloxy-naphtalèn-1-ol,
- le 4-benzyloxy-8-méthoxy-naphtalèn-1-ol,
- le 4-(2,2,2-trifluoroéthoxy) -naphtalèn-1-ol,
- le 4-(2-bromo)éthoxy-naphtalèn-1-ol,
- le 4-(2-bromo)éthoxy-5-méthoxy-naphtalèn-1-ol,
- le 4-(2-bromo)éthoxy-8-méthoxy-naphtalèn-1-ol
- le 4-(2-chloro)éthoxy-naphtalèn-1-ol,
- le 4-(2-chloro)éthoxy-8-méthoxy-naphtalèn-1-ol
- le 4-(2-méthoxy)éthoxy-naphtalèn-1-ol
- le 4-(1,4,7-trioxaheptyl)-naphtalèn-1-ol,
- le 4-(1,4,7-trioxaoctyl)-naphtalèn-1-ol,
- le 4-(1,4,7,10-tétraoxadécyl)-naphtalèn-1-ol,
- l'acide (4-hydroxy-1-naphtalènyl)-oxy acétique,
- le 4-méthoxy-naphtalèn-1-ol,
- le 4-méthoxy-5-chloro-naphtalèn-1-ol,
- le 4-méthoxy-5-chloro-8-benzyloxy-naphtalèn-1-ol,
- le 4,8-diméthoxy-5-chloro-naphtalèn-1-ol,
- le 4-méthoxy-5-méthyl-naphtalèn-1-ol,
- le 4-méthoxy-5-benzyloxy-naphtalèn-1-ol,
- le 4-méthoxy-5-benzyloxy-7-méthyl-naphtalèn-1-ol,
- le 4-méthoxy-5-hydroxy-naphtalèn-1-ol,
- le 4-méthoxy-5-hydroxy-7-méthyl-naphtalèn-1-ol,
- le 4-méthoxy-5-isopropyloxy-naphtalèn-1-ol,
- le 4,5-diméthoxy-naphtalèn-1-ol,
- le 4,5-diméthoxy-6-benzyloxy-naphtalèn-1-ol,
- le 4,5-diméthoxy-7-méthyl-naphtalèn-1-ol,
- le 4,5-diméthoxy-8-chloro-naphtalèn-1-ol,
- le 4-méthoxy-6-méthyl-naphtalèn-1-ol,
- le 4-méthoxy-6-méthyl-7-acétyl-8-hydroxy-naphtalèn-1-ol,
- le 4-méthoxy-6,7-diméthyl-naphtalèn-1-ol,
- le 4-méthoxy-6-méthyl-8-benzyloxy-naphtalèn-1-ol,
- le 4-méthoxy-6-méthyl-8-hydroxy-naphtalèn-1-ol,
- le 4,8-diméthoxy-6-méthyl-naphtalèn-1-ol,
- le 4-méthoxy-6-éthoxy-naphtalèn-1-ol,
- le 4-méthoxy-6,7-diéthoxy-naphtalèn-1-ol,
- le 4-méthoxy-7-méthyl-naphtalèn-1-ol,
- le 4,8-diméthoxy-7-benzyloxy-naphtalèn-1-ol,
- le 4-méthoxy-7-éthoxy-naphtalèn-1-ol,
- le 4-méthoxy-8-chloro-naphtalèn-1-ol,
- le 4-méthoxy-8-méthyl-naphtalèn-1-ol,
- le 4-méthoxy-8-benzyloxy-naphtalèn-1-ol,
- le 4-méthoxy-8-hydroxy-naphtalèn-1-ol,
- le 4-méthoxy-8-isopropyloxy-naphtalèn-1-ol,
- le 4,8-diméthoxy-naphtalèn-1-ol,
- le 4-éthoxy-naphtalèn-1-ol,
- le 4-propyloxy-naphtalèn-1-ol,
- le 4-isopropyloxy-naphtalèn-1-ol,
- le 4-butoxy-naphtalèn-1-ol,
- le 4-isobutoxy-naphtalèn-1-ol,
- le 4-*sec*-butoxy-naphtalèn-1-ol,
- le 4-isoamoxy-naphtalèn-1-ol,
- le 4-bis-(2-chloro-isopropyloxy)-naphtalèn-1-ol,
- le 4-cyclohexyloxy-naphtalèn-1-ol,
- le 4-octyloxy-naphtalèn-1-ol,
- le 4-(2-chloropropoxy)-naphtalèn-1-ol,
- l'isopropylidèn-4,5-dioxy-naphtalèn-1-ol,
- le 5-méthoxy-naphtalèn-1-ol,
- le 5,8-diméthoxy-6-méthyl-naphtalèn-1-ol,
- le 5,8-diméthoxy-6,7-dichloro-naphtalèn-1-ol,
- le 5,8-diméthoxy-7-méthyl-naphtalèn-1-ol,
- le 5,8-diacétoxy-naphtalèn-1-ol,
- le 8-méthoxy-naphtalèn-1-ol.

Parmi les composés de formule (I) conformes à l'invention cités ci-dessus, on préfère tout particulièrement :
- le 4-méthoxy-naphtalèn-1-ol,
- le 4-éthoxy-naphtalèn-1-ol,
- le 4-isopropyloxy-naphtalèn-1-ol, et
- le 4,8-diméthoxy-naphtalèn-1-ol.

Le ou les composés de formule (I) conformes à l'invention représentent de préférence de 0,001 à 5% en poids par rapport au poids total de la composition B1 et encore plus préférentiellement de 0,01 à 2 % en poids de ce poids.

Le pH de la composition A est généralement compris entre 3 et 9 environ, il varie de préférence entre 6 et 8 environ, et encore plus préférentiellement il est égal à 7 environ.

Le pH de la composition B doit être supérieur ou égal à 6 et est de préférence compris entre 7 et 11.

Ainsi, le pH des compositions B1 et B2 est ajusté de manière telle qu'après mélange, juste avant emploi, de la composition B1 avec la composition B2 dans un rapport pondéral variant de préférence de 0,5 à 5 et encore plus préférentiellement de 1 à 3, le pH de la composition B résultante est supérieur ou égal à 6 et de préférence compris entre 7 et 11.

Le pH des compositions A, B1, B2 et B peut être ajusté à la valeur désirée au moyen d'agents alcalinisants ou éventuellement acidifiants habituellement utilisés en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 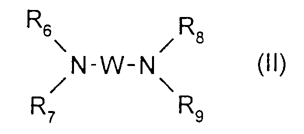 dans laquelle W est un reste propylène éventuellement substitué par un groupement hydroxyle ou un radical alkyle en C₁-C₄ ; R₆, R₇, R₈ et R₉, identiques ou différents, représentent un atome d'hydrogène, un radical alkyle en C₁-C₄ ou hydroxyalkyle en C₁-C₄.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Selon une forme de réalisation particulière de l'invention, la composition B1 peut contenir, en plus des composés de formule (I) conformes à l'invention, une ou plusieurs bases d'oxydation.

La ou les bases d'oxydation éventuelles sont choisies parmi les bases d'oxydation utilisées de façon classique pour la teinture d'oxydation des fibres kératiniques, et sont de préférence choisies parmi les paraphénylènediamines, les bis-phénylalkylènediamines, les para-aminophénols, les ortho-aminophénols, les bases hétérocycliques, et leurs sels d'addition avec un acide.

Lorsqu'elles sont présentes, la ou les bases d'oxydation représentent de préférence de 0,001 à 7 % en poids environ du poids total de la composition B1 et encore plus préférentiellement de 0,01 à 2 % en poids environ de ce poids.

La composition B1 utilisée selon le procédé de l'invention peut également renfermer un ou plusieurs coupleurs et/ou un ou plusieurs colorants directs de façon à faire varier ou enrichir en reflets les nuances obtenues avec les composés de formule (I) conformes à l'invention.

Les coupleurs utilisables dans la composition B1 mise en oeuvre dans le procédé conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, et leurs sels d'addition avec un acide.

Lorsqu'ils sont présents ces coupleurs représentent de préférence de 0,001 à 7 % en poids environ du poids total de la composition B1 et encore plus préférentiellement de 0,01 à 2 % en poids environ de ce poids.

Les sels d'addition avec un acide des bases d'oxydation et/ou des coupleurs utilisables dans la composition B1 de l'invention sont notamment choisis parmi les chlorhydrates, les bromhydrates, les sulfates, les tartrates, les lactates et les acétates.

L'agent oxydant présent dans la composition B2 telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les iodates et periodates, les oxydes de métaux tels que l'oxyde d'argent, les chlorures métalliques tels que le chlorure ferrique, le ferricyanure, les bromates de métaux alcalins, les persels tels que les perborates et persulfates. On utilise de préférence du peroxyde d'hydrogène à une concentration comprise entre 0,03 et 100 volumes (soit entre 0,01 et 30 % en poids) et encore plus préférentiellement entre 2 et 20 volumes (soit entre 0,6 et 6 % en poids).

Le milieu approprié pour la teinture (ou support) des compositions A, B1 et B2 est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther de diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de chaque compositions dans laquelle ils sont contenus, et encore plus préférentiellement entre 5 et 30 % en poids environ de ce poids.

Les compositions A et/ou B1 et/ou B2, utilisées selon le procédé conforme à l'invention, peuvent également renfermer divers adjuvants utilisés classiquement dans les compositions pour la teinture des cheveux, tels que des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la mise en oeuvre selon l'invention des compositions A, B1 et B2 ne soient pas, ou substantiellement pas, altérées par la ou les adjonctions envisagées.

La composition A utilisée selon le procédé conforme à l'invention peut se présenter sous la forme d'un liquide plus ou moins épaissi et notamment sous la forme de lotion ou de shampooing.

La composition B résultant du mélange extemporané des compositions B1 et B2, utilisée selon le procédé conforme à l'invention, peut se présenter sous des formes diverses, telles que sous forme de liquides, de crèmes, de gels, de shampooings ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme une composition A contenant, dans un milieu approprié pour la teinture, au moins un sel de manganèse et/ou au moins un complexe de manganèse, un deuxième compartiment renferme une composition B1 contenant, dans un milieu approprié pour la teinture, au moins un composé de formule (I) telle que définie précédemment et un troisième compartiment renferme une composition B2 contenant, dans un milieu approprié pour la teinture au moins un agent oxydant tel que défini précédemment, le pH des compositions B1 et B2 étant ajusté de manière telle qu'après mélange de la composition B1 avec la composition B2 dans un rapport pondéral variant de préférence de 0,5 à 5 et encore plus préférentiellement de 1 à 3, le pH de la composition B résultante est supérieur ou égal à 6 et de préférence compris entre 7 et 11. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLE 1 DE TEINTURE

On a préparé les compositions A, B1 et B2 conformes à l'invention suivantes (teneurs en grammes) :

### Composition A :

| | |
|---|---|
| - Bis-(hexafluorophosphate) de bis-(octahydro-1,4,7-triméthyl-1H-1, 4, 7-triazonine-N1, N4, N7) tri-µ-oxo di-manganèse (complexe de manganèse) | 0,001 g |
| - Eau déminéralisée q.s.p. | 100 g |

### Composition B1

| | |
|---|---|
| - Alcool oléique polyglycérolé à 2 moles de glycérol | 4,0 g |
| - Alcool oléique polyglycérolé à 4 moles de glycérol, à 78 % de matières actives (M.A.) | 5,69 g M.A. |
| - Acide oléique | 3,0 g |
| - Amine oléique à 2 moles d'oxyde d'éthylène vendue sous la dénomination commerciale ETHOMEEN O12 par la société AKZO | 7,0 g |
| - Laurylamino succinamate de diéthylaminopropyle, sel de sodium à 55 % de M.A. | 3,0 g M.A. |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse, à 35 % de M.A. | 0,455 g M.A. |
| - Acétate d'ammonium | 0,8 g |
| - Antioxydant, séquestrant | q.s. |
| - Parfum, conservateur | q.s. |
| - Ammoniaque à 20 % de NH₃ | 10,0 g |
| - **4-méthoxy-naphtalèn-1-ol (6.10**^{**-3**} **mole)** | **1,04 g** |
| - Eau déminéralisée q.s.p. | 100,0 g |

### Composition B2

Solution de peroxyde d'hydrogène à 20 volumes (6% en poids).

La composition A a été appliquée sur des mèches de cheveux gris naturels à 90 % de blancs pendant 30 secondes, à raison de 2 g de composition A pour 1 g de cheveu à traiter, puis les mèches ont été essorées.

Juste avant application, la composition B1 a été mélangée poids pour poids avec la solution B2 de peroxyde d'hydrogène, pour obtenir une composition B.

La composition B résultante a ensuite été appliquée sur les mèches de cheveux pendant 30 minutes. Les cheveux ont ensuite été rincés, lavés au shampooing puis séchés.

Les mèches de cheveux ont été teintes dans une nuance bleue.

### EXEMPLES 2 à 5 DE TEINTURE

On a préparé les compositions A, B1 et B2 conformes à l'invention suivantes (teneurs en grammes) :

### Composition A :

| | |
|---|---|
| - Bis-(hexafluorophosphate) de bis-(octahydro-1,4,7-triméthyl-1H-1, 4, 7-triazonine-N1, N4, N7) tri-µ-oxo di-manganèse (complexe de manganèse) | 0,001 g |
| - Eau déminéralisée q.s.p. | 100 g |

### Compositions B1

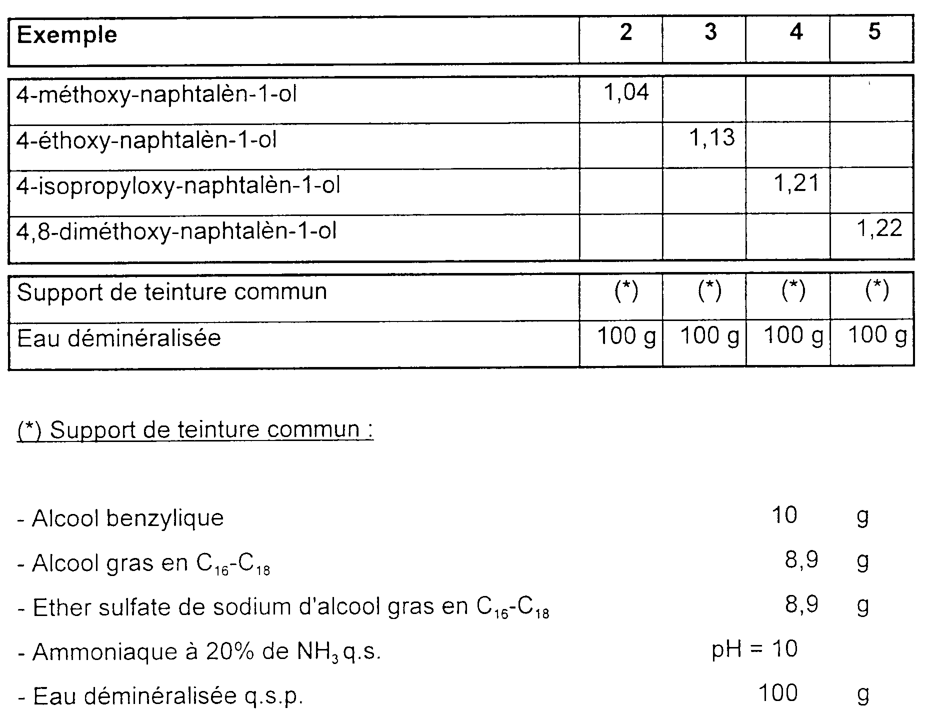

### Composition B2

Solution de peroxyde d'hydrogène à 20 volumes (6% en poids).

La composition A a été appliquée sur des mèches de cheveux gris naturels à 90 % de blancs pendant 30 secondes, à raison de 2 g de composition A pour 1 g de cheveu à traiter, puis les mèches ont été essorées.

Juste avant application, chaque composition B1 a été mélangée poids pour poids avec la solution B2 de peroxyde d'hydrogène, pour obtenir les compositions B correspondantes.

Chaque composition B résultante a ensuite été appliquée sur les mèches de cheveux pendant 30 minutes. Les cheveux ont ensuite été rincés, lavés au shampooing puis séchés.

Les mèches de cheveux ont été teintes dans les nuances figurant dans le tableau ci-après :

| **Exemple** | **Nuance obtenue** |
|---|---|
| **2** | Bleu |
| **3** | Bleu |
| **4** | Bleu |
| **5** | Jaune orangé |

### EXEMPLES 6 à 8 DE TEINTURE

On a préparé les compositions A, B1 et B2 conformes à l'invention suivantes (teneurs en grammes) :

### Composition A :

| | |
|---|---|
| - Bis-(hexafluorophosphate) de bis-(octahydro-1,4,7-triméthyl-1H-1, 4, 7-triazonine-N1, N4, N7) tri-µ-oxo di-manganèse (complexe de manganèse) | 0,0001 g |
| - Eau déminéralisée q.s.p. | 100 g |

### Compositions B1

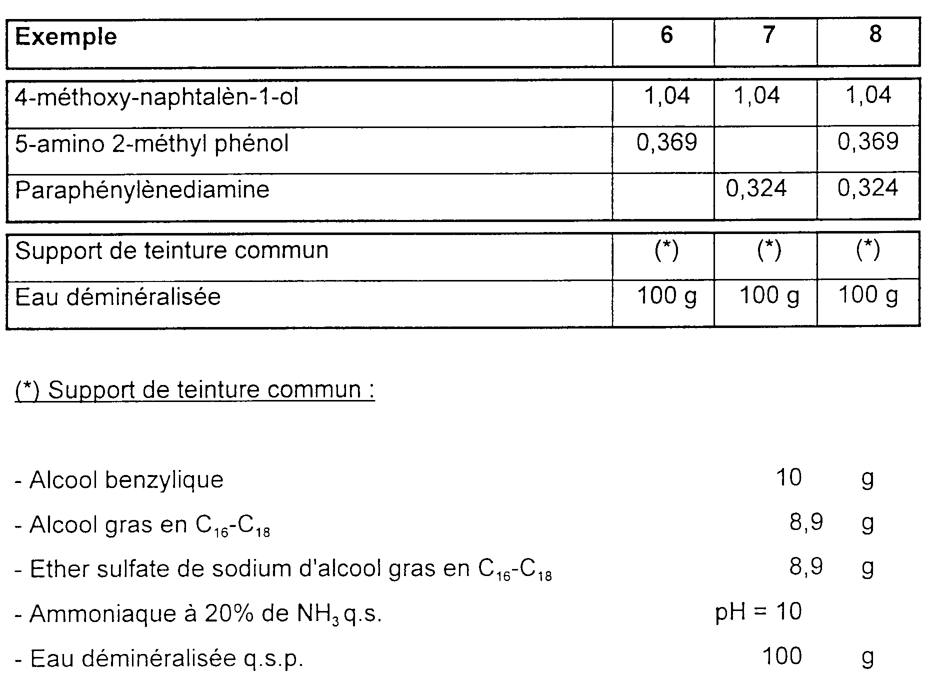

### Composition B2

Solution de peroxyde d'hydrogène à 20 volumes (6% en poids).

La composition A a été appliquée sur des mèches de cheveux gris naturels à 90 % de blancs pendant 30 secondes, à raison de 2 g de composition A pour 1 g de cheveu à traiter, puis les mèches ont été essorées.

Juste avant application, chaque composition B1 a été mélangée poids pour poids avec la solution B2 de peroxyde d'hydrogène, pour obtenir les compositions B correspondantes.

Chaque composition B résultante a ensuite été appliquée sur les mèches de cheveux pendant 30 minutes. Les cheveux ont ensuite été rincés, lavés au shampooing puis séchés.

Les mèches de cheveux ont été teintes dans les nuances figurant dans le tableau ci-après :

| **Exemple** | **Nuance obtenue** |
|---|---|
| **6** | Bleu |
| **7** | Violet intense |
| **8** | Aubergine intense |

## Revendications

1. Procédé de teinture d'oxydation en deux temps des fibres kératiniques et en particulier des fibres kératiniques humaines telles que les cheveux, caractérisé par le fait qu'on applique sur ces fibres :
- dans un premier temps, au moins une composition A contenant, dans un milieu approprié pour la teinture, au moins un sel de manganèse et/ou au moins un complexe de manganèse,
- et dans un deuxième temps, au moins une composition B présentant un pH supérieur ou égal à 6 et résultant du mélange extemporané :
a) d'une composition B1 contenant, dans un milieu approprié pour la teinture, au moins un 1-naphtol 4-substitué de formule (I) suivante : 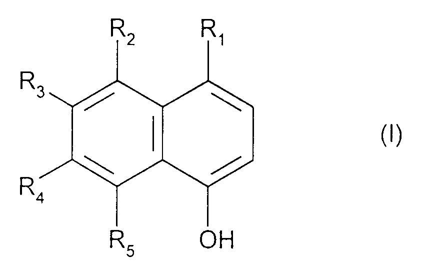 dans laquelle :
R₁ représente un atome d'halogène tel que le brome, le chlore, l'iode ou le fluor; un radical benzyle ; un radical benzyloxy ; un radical alcoxy linéaire, ramifié ou cyclique en C₁-C₂₉ pouvant être substitué par un ou plusieurs atomes d'halogène et/ou interrompu par un ou plusieurs hétéroatomes ; un radical acyloxy en C₁-C₂₉ ou un radical alcoxycarboxylique en en C₁-C₂₉ ;
R₂, R₃, R₄ et R₅, identiques ou différents, représentent un atome d'hydrogène, un atome d'halogène tel que le brome, le chlore, l'iode ou le fluor, un radical hydroxyle, alkyle linéaire ou ramifié en C₁-C₂₉, alcoxy linéaire ou ramifié en C₁-C₂₉, acyle ou benzyloxy.
b) et d'une composition B2 contenant, dans un milieu approprié pour la teinture, au moins un agent oxydant.

2. Procédé selon la revendication 1, caractérisé par le fait que l'application de la composition A est séparée de l'application de la composition B par une étape intermédiaire d'essorage et/ou de rinçage.

3. Procédé selon la revendication 1 ou 2, caractérisé par le fait que la composition A est appliquée sur les fibres kératiniques avec un temps de pose compris entre 5 secondes et 10 minutes, la composition B est appliquée sur les fibres avec un temps de pose compris entre 30 secondes et 45 minutes.

4. Procédé selon la revendication 3, caractérisé par le fait que la composition A est appliquée sur les fibres kératiniques avec un temps de pose compris entre 30 secondes et 5 minutes, la composition B est appliquée sur les fibres avec un temps de pose compris entre 3 et 30 minutes.

5. Procédé selon l'une quelconque des revendications précédentes, caractérisé par le fait que le ou les sels de manganèse n'ont pas d'activité oxydante propre et que le manganèse présente un degré d'oxydation égal à 2 ou à 3.

6. Procédé selon la revendication 5, caractérisé par le fait que les sels de manganèse sont choisis parmi les sels hydrosolubles, minéraux ou organiques du manganèse.

7. Procédé selon la revendication 6, caractérisé par le fait que les sels de manganèse sont choisis parmi le dichlorure de manganèse, le trichlorure de manganèse, les sulfates de manganèse, les nitrates de manganèse, le diacétate de manganèse, les carbonates de manganèse, les dihydrogénocarbonates de manganèse, l'acétylacétonate de manganèse, le triacétate de manganèse, le lactate de manganèse, le formate de manganèse, l'acétyl méthionate de manganèse, le gluconate de manganèse, les complexes de manganèse, et leurs hydrates.

8. Procédé selon l'une quelconque des revendications 1 à 4, caractérisé par le fait que le ou les complexes de manganèse n'ont pas d'activité oxydante propre et sont choisis parmi les complexes de manganèse dans lesquels le manganèse présente un degré d'oxydation égal à 4.

9. Procédé selon la revendication 8, caractérisé par le fait que le complexe de manganèse est le bis-(hexafluorophosphate) de bis-(octahydro-1,4,7-triméthyl-1H-1, 4, 7-triazonine-N1, N4, N7) tri-µ-oxo di-manganèse.

10. Procédé selon l'une quelconque des revendications précédentes, caractérisé par le fait que le ou les sels de manganèse et/ou le ou les complexes de manganèse sont présents à une concentration comprise entre 0,0001 et 1 % en poids d'équivalents métal par rapport au poids total de la composition A.

11. Procédé selon l'une quelconque des revendications précédentes, caractérisé par le fait que les composés de formule (I) sont choisis parmi :
- le 4-benzyl-naphtalèn-1-ol,
- le 4-chloro naphtalèn-1-ol,
- le 4-chloro-5,8-diméthoxy-6-méthyl- naphtalèn-1-ol,
- le 4-chloro-5,8-diméthoxy- naphtalèn-1-ol,
- le 4-acétoxy-5-chloro-6-méthyl-7-acétyl-8-hydroxy-naphtalèn-1-ol,
- le 4-acétoxy-6-méthyl-7-acétyl-8-hydroxy-naphtalèn-1-ol,
- le 4-acétoxy-8-benzyloxy-naphtalèn-1-ol,
- le 4-benzyloxy-naphtalèn-1-ol,
- le 4,8-dibenzyloxy-6-méthyl-naphtalèn-1-ol,
- le 4,8-dibenzyloxy-naphtalèn-1-ol,
- le 4-benzyloxy-8-(2-chloro)éthoxy-naphtalèn-1-ol,
- le 4-benzyloxy-8-isopropyloxy-naphtalèn-1-ol,
- le 4-benzyloxy-8-méthoxy-naphtalèn-1-ol,
- le 4-(2,2,2-trifluoroéthoxy)-naphtalèn-1-ol,
- le 4-(2-bromo)éthoxy-naphtalèn-1-ol,
- le 4-(2-bromo)éthoxy-5-méthoxy-naphtalèn-1-ol,
- le 4-(2-bromo)éthoxy-8-méthoxy-naphtalèn-1-ol,
- le 4-(2-chloro)éthoxy-naphtalèn-1-ol,
- le 4-(2-chloro)éthoxy-8-méthoxy-naphtalèn-1-ol
- le 4-(2-méthoxy)éthoxy-naphtalèn-1-ol
- le 4-(1,4,7-trioxaheptyl)-naphtalèn-1-ol,
- le 4-(1,4,7-trioxaoctyl)-naphtalèn-1-ol,
- le 4-(1,4,7,10-tétraoxadécyl)-naphtalèn-1-ol,
- l'acide (4-hydroxy-1-naphtalènyl)-oxy acétique,
- le 4-méthoxy-naphtalèn-1-ol,
- le 4-méthoxy-5-chloro-naphtalèn-1-ol,
- le 4-méthoxy-5-chloro-8-benzyloxy-naphtalèn-1-ol,
- le 4,8-diméthoxy-5-chloro-naphtalèn-1-ol,
- le 4-méthoxy-5-méthyl-naphtalèn-1-ol,
- le 4-méthoxy-5-benzyloxy-naphtalèn-1-ol,
- le 4-méthoxy-5-benzyloxy-7-méthyl-naphtalèn-1-ol,
- le 4-méthoxy-5-hydroxy-naphtalèn-1-ol,
- le 4-méthoxy-5-hydroxy-7-méthyl-naphtalèn-1-ol,
- le 4-méthoxy-5-isopropyloxy-naphtalèn-1-ol,
- le 4,5-diméthoxy-naphtalèn-1-ol,
- le 4,5-diméthoxy-6-benzyloxy-naphtalèn-1-ol,
- le 4,5-diméthoxy-7-méthyl-naphtalèn-1-ol,
- le 4,5-diméthoxy-8-chloro-naphtalèn-1-ol,
- le 4-méthoxy-6-méthyl-naphtalèn-1-ol,
- le 4-méthoxy-6-méthyl-7-acétyl-8-hydroxy-naphtalèn-1-ol,
- le 4-méthoxy-6,7-diméthyl-naphtalèn-1-ol,
- le 4-méthoxy-6-méthyl-8-benzyloxy-naphtalèn-1-ol,
- le 4-méthoxy-6-méthyl-8-hydroxy-naphtalèn-1-ol,
- le 4,8-diméthoxy-6-méthyl-naphtalèn-1-ol,
- le 4-méthoxy-6-éthoxy-naphtalèn-1-ol,
- le 4-méthoxy-6,7-diéthoxy-naphtalèn-1-ol,
- le 4-méthoxy-7-méthyl-naphtalèn-1-ol,
- le 4,8-diméthoxy-7-benzyloxy-naphtalèn-1-ol,
- le 4-méthoxy-7-éthoxy-naphtalèn-1-ol,
- le 4-méthoxy-8-chloro-naphtalèn-1-ol,
- le 4-méthoxy-8-méthyl-naphtalèn-1-ol,
- le 4-méthoxy-8-benzyloxy-naphtalèn-1-ol,
- le 4-méthoxy-8-hydroxy-naphtalèn-1-ol,
- le 4-méthoxy-8-isopropyloxy-naphtalèn-1-ol,
- le 4,8-diméthoxy-naphtalèn-1-ol,
- le 4-éthoxy-naphtalèn-1-ol,
- le 4-propyloxy-naphtalèn-1-ol,
- le 4-isopropyloxy-naphtalèn-1-ol,
- le 4-butoxy-naphtalèn-1-ol,
- le 4-isobutoxy-naphtalèn-1-ol,
- le 4-*sec*-butoxy-naphtalèn-1-ol,
- le 4-isoamoxy-naphtalèn-1-ol,
- le 4-bis-(2-chloro-isopropyloxy)-naphtalèn-1-ol,
- le 4-cyclohexyloxy-naphtalèn-1-ol,
- le 4-octyloxy-naphtalèn-1-ol,
- le 4-(2-chloropropoxy)-naphtalèn-1-ol,
- l'isopropylidèn-4,5-dioxy-naphtalèn-1-ol,
- le 5-méthoxy-naphtalèn-1-ol,
- le 5,8-diméthoxy-6-méthyl-naphtalèn-1-ol,
- le 5,8-diméthoxy-6,7-dichloro-naphtalèn-1-ol,
- le 5,8-diméthoxy-7-méthyl-naphtalèn-1-ol,
- le 5,8-diacétoxy-naphtalèn-1-ol,
- le 8-méthoxy-naphtalèn-1-ol.

12. Procédé selon la revendication 11, caractérisé par le fait que les composés de formule (I) sont choisis parmi :
- le 4-méthoxy-naphtalèn-1-ol,
- le 4-éthoxy-naphtalèn-1-ol,
- le 4-isopropyloxy-naphtalèn-1-ol, et
- le 4,8-diméthoxy-naphtalèn-1-ol.

13. Procédé selon l'une quelconque des revendications précédentes, caractérisé par le fait que le ou les composés de formule (I) représentent de 0,001 à 5% en poids par rapport au poids total de la composition B1.

14. Procédé selon l'une quelconque des revendications précédentes, caractérisé par le fait que le pH de la composition A est compris entre 3 et 9.

15. Procédé selon l'une quelconque des revendications précédentes, caractérisé par le fait que le pH de la composition B est supérieur ou égal à 6.

16. Procédé selon l'une quelconque des revendications précédentes, caractérisé par le fait que la composition B1 contient une ou plusieurs bases d'oxydation et/ou un ou plusieurs coupleurs et/ou un ou plusieurs colorants directs.

17. Procédé selon l'une quelconque des revendications précédentes, caractérisé par le fait que les sels d'addition avec un acide des bases d'oxydation et /ou des coupleurs utilisables dans la composition B1 sont choisis parmi les chlorhydrates, les bromhydrates, les sulfates et les tartrates, les lactates et les acétates.

18. Procédé selon l'une quelconque des revendications précédentes, caractérisé par le fait que l'agent oxydant présent dans la composition B2 est choisi parmi le peroxyde d'hydrogène, le peroxyde d'urée, les iodates et periodates, les oxydes de métaux, les chlorures métalliques, le ferricyanure, les bromates de métaux alcalins et les persels tels que les perborates et persulfates.

19. Procédé selon la revendication 18, caractérisé par le fait que l'agent oxydant est le peroxyde d'hydrogène.

20. Procédé selon l'une quelconque des revendications précédentes, caractérisé par le fait que le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

21. Dispositif à plusieurs compartiments ou "kit" de teinture à plusieurs compartiments, caractérisé par le fait qu'un premier compartiment renferme une composition A contenant, dans un milieu approprié pour la teinture, au moins un sel de manganèse et/ou au moins un complexe de manganèse, un deuxième compartiment renferme une composition B1 contenant, dans un milieu approprié pour la teinture, au moins un composé de formule (I) tel que défini dans l'une quelconque des revendications 1, 11 ou 12 et un troisième compartiment renferme une composition B2 contenant, dans un milieu approprié pour la teinture au moins un agent oxydant, le pH des compositions B1 et B2 étant ajusté de manière telle qu'après mélange de la composition B1 avec la composition B2 dans un rapport pondéral variant de 0,5 à 5, le pH de la composition B résultante est supérieur ou égal à 6.

## Claims

1. Process for the two-stage oxidation dyeing of keratin fibres, and in particular human keratin fibres such as the hair, characterized in that:
- in a first stage, at least one composition A containing, in a medium which is suitable for dyeing, at least one manganese salt and/or at least one manganese complex, and
- in a second stage, at least one composition B having a pH of greater than or equal to 6, and resulting from the extemporaneous mixing:
a) of a composition B1 containing, in a medium which is suitable for dyeing, at least one 4-substituted 1-napththol of formula (1) below: 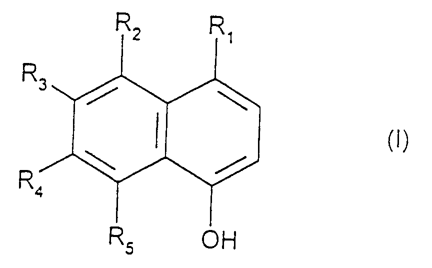 in which:
R₁ represents a halogen atom such as bromine, chlorine, iodine or fluorine; a benzyl radical; a benzyloxy radical; a linear, branched or cyclic C₁-C₂₉ alkoxy radical which may be substituted with one or more halogen atoms and/or interrupted by one or more hetero atoms; a C₁-C₂₉ acyloxy radical or a C₁-C₂₉ alkoxycarboxylic radical;
R₂, R₃, R₄ and R₅, which may be identical or different, represent a hydrogen atom, a halogen atom such as bromine, chlorine, iodine or fluoroine or a hydroxyl, linear or branched C₁-C₂₉ alkyl, linear or branched C₁-C₂₉ alkoxy, acyl or benzyloxy radical,
b) and a composition B2 containing, in a medium which is suitable for dyeing, at least one oxidizing agent, are applied to these fibres.

2. Process according to Claim 1, characterized in that the application of composition A is separated from the application of composition B by an intermediate towel-drying and/or rinsing step.

3. Process according to Claim 1 or 2, characterized in that composition A is applied to the keratin fibres for an exposure time of between 5 seconds and 10 minutes, and composition B is applied to the fibres for an exposure time of between 30 seconds and 45 minutes.

4. Process according to Claim 3, characterized in that composition A is applied to the keratin fibres for an exposure time of between 30 seconds and 5 mintues and composition B is applied to the fibres for an exposure time of between 3 and 30 minutes.

5. Process according to any one of the preceding claims, characterized in that the manganese salt(s) has (have) no intrinsic oxidizing activity and the manganese has a degree of oxidation equal to 2 or 3.

6. Process accoding to Claim 5, characterized in that the manganese salts are chosen from organic or inorganic, water-soluble manganese salts.

7. Process according to Claim 6, characterized in that the manganese salts are chosen from manganese dichloride, manganese trichloride, manganese sulphates, manganese nitrates, manganese diacetate, manganese carbonates, manganese dihydrogen carbonates, manganese acetylacetonate, manganese triacetate, manganese lactate, manganese formate, manganese acetylmethionate and manganese gluconate, and manganese complexes, and hydrates thereof.

8. Process according to any one of Claims 1 to 4, characterized in that the manganese complex(es) has (have) no intrinsic oxidizing activity and is (are) chosen from manganese complexes in which the manganese has a degree of oxidation equal to 4.

9. Process according to Claim 8, characterized in that the manganese complex is bis(octahydro-1,4,7-trimethyl-1H-1,4,7-triazinone-N1,N4,N7)tri-µ-oxodi-manganese bis(hexafluorophosphate).

10. Process according to any one of the preceding claims, characterized in that the manganese salt(s) and/or the manganese complex(es) is (are) present in a concentration of between 0.0001 and 1% by weight of metal equivalents relative to the total weight of composition A.

11. Process according to any one of the preceding claims, characterized in that the compounds of formula (I) are chosen from:
- 4-benzylnaphth-1-ol,
- 4-chloronaphth-1-ol,
- 4-chloro-5,8-dimethoxy-6-methylnaphth-1-ol,
- 4-chloro-5,8-dimethoxynaphth-1-ol,
- 4-acetoxy-5-chloro-6-methyl-7-acetyl-8-hydroxynaphth-1-ol,
- 4-acetoxy-6-methyl-7-acetyl-8-hydroxynapth-1-ol,
- 4-acetoxy-8-benzyloxynaphth-1-ol,
- 4-benzyloxynaphth-1-ol,
- 4,8-dibenzyloxy-6-methylnaphth-1-ol,
- 4,8-dibenzyloxynaphth-1-ol,
- 4-benzyloxy-8-(2-chloro)ethoxynaphth-1-ol,
- 4-benzyloxy-8-isopropyloxynaphth-1-ol,
- 4-benzyloxy-8-methoxynaphth-1-ol,
- 4-(2,2,2-trifluoroethoxy)naphth-1-ol,
- 4-(2-bromo)ethoxynaphth-1-ol,
- 4-(2-bromo)ethoxy-5-methoxynaphth-1-ol,
- 4-(2-bromo)ethoxy-8-methoxynaphth-1-ol,
- 4-(2-chloro)ethoxynaphth-1-ol,
- 4-(2-chloro)ethoxy-8-methoxynaphth-1-ol,
- 4-(2-methoxy)ethoxynaphth-1-ol,
- 4-(1,4,7-trioxaheptyl)naphth-1-ol,
- 4-(1,4,7-trioxaoctyl)naphth-1-ol,
- 4-(1,4,7,10-tetraoxadecyl)naphth-1-ol,
- (4-hydroxy-1-naphthyl)oxyacetic acid,
- 4-methoxynaphth-1-ol,
- 4-methoxy-5-chloronaphth-1-ol,
- 4-methoxy-5-chloro-8-benzyloxynaphth-1-ol,
- 4,8-dimethoxy-5-chloronaphth-1-ol,
- 4-methoxy-5-methylnaphth-1-ol,
- 4-methoxy-5-benzyloxynaphth-1-ol,
- 4-methoxy-5-benzyloxy-7-methylnaphth-1-ol,
- 4-methoxy-5-hydroxynaphth-1-ol,
- 4-methoxy-5-hydroxy-7-methylnaphth-1-ol,
- 4-methoxy-5-isopropyloxynaphth-1-ol,
- 4,5-dimethoxynaphth-1-ol,
- 4,5-dimethoxy-6-benzyloxynaphth-1-ol,
- 4,5-dimethoxy-7-methylnaphth-1-ol,
- 4,5-dimethoxy-8-chloronaphth-1-ol,
- 4-methoxy-6-methylnaphth-1-ol,
- 4-methoxy-6-methyl-7-acetyl-8-hydroxynaphth-1-ol,
- 4-methoxy-6,7-dimethylnaphth-1-ol,
- 4-methoxy-6-methyl-8-benzyloxynaphth-1-ol,
- 4-methoxy-6-methyl-8-hydroxynaphth-1-ol,
- 4,8-dimethoxy-6-methylnaphth-1-ol,
- 4-methoxy-6-ethoxynaphth-1-ol,
- 4-methoxy-6,7-diethoxynaphth-1-ol,
- 4-methoxy-7-methylnaphth-1-ol,
- 4,8-dimethoxy-7-benzyloxynaphth-1-ol,
- 4-methoxy-7-ethoxynaphth-1-ol,
- 4-methoxy-8-chloronaphth-1-ol,
- 4-methoxy-8-methylnaphth-1-ol,
- 4-methoxy-8-benzyloxynaphth-1-ol,
- 4-methoxy-8-hydroxynaphth-1-ol,
- 4-methoxy-8-isopropyloxynaphth-1-ol,
- 4,8-dimethoxynaphth-1-ol,
- 4-ethoxynaphth-1-ol,
- 4-propyloxynaphth-1-ol,
- 4-isopropyloxynaphth-1-ol,
- 4-butoxynaphth-1-ol,
- 4-isobutoxynaphth-1-ol,
- 4-*sec*-butoxynaphth-1-ol,
- 4-isoamoxynaphth-1-ol,
- 4-bis(2-chloroisopropyloxy)naphth-1-ol,
- 4-cyclohexyloxynaphth-1-ol,
- 4-octyloxynaphth-1-ol,
- 4-(2-chloropropoxy)naphth-1-ol,
- isopropylidene-4,5-dioxynaphth-1-ol,
- 5-methoxynaphth-1-ol,
- 5,8-dimethoxy-6-methylnaphth-1-ol,
- 5,8-dimethoxy-6,7-dichloronaphth-1-ol,
- 5,8-dimethoxy-7-methylnaphth-1-ol,
- 5,8-diacetoxynaphth-1-ol,
- 8-methoxynaphth-1-ol.

12. Process according to Claim 11, characterized in that the compounds of formula (I) are chosen from:
- 4-methoxynaphth-1-ol,
- 4-ethoxynaphth-1-ol,
- 4-isopropyloxynaphth-1-ol, and
- 4,8-dimethoxynaphth-1-ol.

13. Process according to any one of the preceding claims, characterized in that the compound(s) of formula (I) represent(s) from 0.001 to 5% by weight relative to the total weight of composition B1.

14. Process according to any one of the preceding claims, characterized in that the pH of compositions A is between 3 and 9.

15. Process according to any one of the preceding claims, characterized in that the pH of composition B is greater than or equal to 6.

16. Process according to any one of the preceding claims, characterized in that composition B1 contains one or more oxidation bases and/or one or more couplers and/or one or more direct dyes.

17. Process according to any one of the preceding claims, characterized in that the addition salts with an acid of the oxidation bases and/or of the couplers which can be used in composition B1 are chosen from the hydrochlorides, hydrobromides, sulphates, tartrates, lactates and acetates.

18. Process according to any one of the preceding claims, characterized in that the oxidizing agent present in composition B2 is chosen from hydrogen peroxide, urea peroxide, iodates and periodates, metal oxides, metal chlorides, ferric cyanide, alkali metal bromates, and persalts such as perborates and persulphates.

19. Process according to Claim 18, characterized in that the oxidizing agent is hydrogen peroxide.

20. Process according to any one of the preceding claims, characterized in that the appropriate dyeing medium (or the support) consists of water or of a mixture of water and at least one organic solvent chosen from C₁-C₄ lower alkanols, glycerol, glycols and glycol ethers, aromatic alcohols, similar products and mixtures thereof.

21. Multi-compartment device or multi-compartment dyeing "kit", characterized in that a first compartment contains a composition A containing, in a medium which is suitable for dyeing, at least one manganese salt and/or at least one manganese complex, a second compartment contains a composition B1 containing, in a medium which is suitable for dyeing, at least one compound of formula (I) as defined in any one of Claims 1, 11 or 12, and a third compartment contains a composition B2 containing, in a medium which is suitable for dyeing, at least one oxidizing agent, the pH of compositions B1 and B2 being adjusted such that after mixing composition B1 with composition B2 in a weight ratio ranging from 0.5 to 5, the pH of the resulting composition B is greater than or equal to 6.

## Patentansprüche

1. Zweistufiges Verfahren zum oxidativen Färben von Keratinfasern und insbesondere menschlichen Keratinfasern, wie dem Haar, dadurch gekennzeichnet, daß auf die Fasern aufgebracht werden:
- in einem ersten Schritt mindestens eine Zusammensetzung A, die in einem zum Färben geeigneten Medium mindestens ein Mangansalz und/oder mindestens einen Mangankomplex enthält, und
- in einem zweiten Schritt mindestens eine Zusammensetzung B, die einen pH-Wert von 6 oder darüber aufweist und hergestellt wird, indem vorab:
a)eine Zusammensetzung B1, die in einem zum Färben geeigneten Medium mindestens ein 4-substituiertes 1-Naphthol der folgenden Formel (I) enthält: 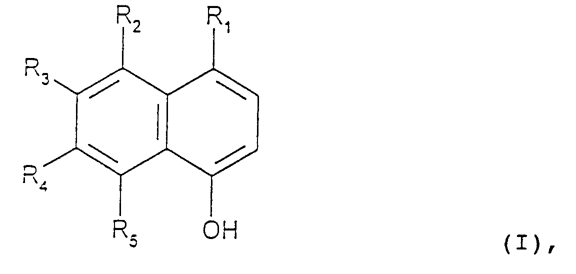 worin bedeuten:
- R₁ ein Halogenatom, wie Brom, Chlor, Iod oder Fluor; eine Benzylgruppe; eine Benzyloxygruppe; eine geradkettige, verzweigte oder cyclische C₁₋₂₉-Alkoxygruppe, die mit einem oder mehreren Halogenatomen substituiert sein kann und/oder ein oder mehrere Heteroatome aufweisen kann; eine C₁₋₂₉-Acyloxygruppe oder eine C₁₋₂₉-Alkoxycarboxygruppe; und
- R₂, R₃, R₄ und R₅, die identisch oder voneinander verschieden sind, ein Wasserstoffatom, ein Halogenatom, wie Brom, Chlor, Iod oder Fluor, eine Hydroxygruppe, eine geradkettige oder verzweigte C₁₋₂₉-Alkylgruppe, eine geradkettige oder verzweigte C₁₋₂₉-Alkoxygruppe, eine Acylgruppe oder eine Benzyloxygruppe;
b)mit einer Zusammensetzung B2, die in einem zum Färben geeigneten Medium mindestens ein Oxidationsmittel enthält,
vermischt wird.

2. Verfahren nach Anspruch 1, dadurch gekennzeichnet, daß nach dem Aufbringen der Zusammensetzung A und vor dem Aufbringen der Zusammensetzung B in einem Zwischenschritt ausgewrungen und/oder gespült wird.

3. Verfahren nach Anspruch 1 oder 2, dadurch gekennzeichnet, daß die Zusammensetzung A mit einer Einwirkzeit im Bereich von 5 Sekunden bis 10 Minuten auf die Keratinfasern aufgebracht wird und die Zusammensetzung B mit einer Einwirkzeit im Bereich von 30 Sekunden bis 45 Minuten auf die Fasern aufgebracht wird.

4. Verfahren nach Anspruch 3, dadurch gekennzeichnet, daß die Zusammensetzung A mit einer Einwirkzeit im Bereich von 30 Sekunden bis 5 Minuten auf die Keratinfasern aufgebracht wird und die Zusammensetzung B mit einer Einwirkzeit im Bereich von 3 bis 30 Minuten auf die Fasern aufgebracht wird.

5. Verfahren nach einem der vorhergehenden Ansprüche, dadurch gekennzeichnet, daß das Mangansalz oder die Mangansalze selbst nicht oxidierend wirken und daß das Mangan eine Oxidationsstufe von 2 oder 3 aufweist.

6. Verfahren nach Anspruch 5, dadurch gekennzeichnet, daß die Mangansalze unter anorganischen oder organischen wasserlöslichen Mangansalzen ausgewählt sind.

7. Verfahren nach Anspruch 6, dadurch gekennzeichnet, daß die Mangansalze ausgewählt sind unter Mangandichlorid, Mangantrichlorid, Mangansulfaten, Mangannitraten, Mangandiacetat, Mangancarbonaten, Mangandihydrogencarbonaten, Manganacetylacetonat, Mangantriacetat, Manganlactat, Manganformiat, Manganacetylmethionat, Mangangluconat, Mangankomplexen und den Hydraten dieser Verbindungen.

8. Verfahren nach einem der Ansprüche 1 bis 4, dadurch gekennzeichnet, daß der Mangankomplex oder die Mangankomplexe selbst nicht oxidierend wirken und unter Mangankomplexen ausgewählt sind, worin das Mangan eine Oxidationsstufe von 4 aufweist.

9. Verfahren nach Anspruch 8, dadurch gekennzeichnet, daß es sich bei dem Mangankomplex um Bis(octahydro-1,4,7-trimethyl-*1H*-1,4,7-triazonin-N1,N4,N7)-tri-µ-oxo-dimangan-bis(hexafluorphosphat) handelt.

10. Verfahren nach einem der vorhergehenden Ansprüche, dadurch gekennzeichnet, daß das Mangansalz oder die Mangansalze und/oder der Mangankomplex oder die Mangankomplexe in einer Konzentration im Bereich von 0,0001 bis 1 Gew.-%, ausgedrückt als Metall, bezogen auf das Gesamtgewicht der Zusammensetzung A, vorliegen.

11. Verfahren nach einem der vorhergehenden Ansprüche, dadurch gekennzeichnet, daß die Verbindungen der Formel (I) ausgewählt sind unter:
- 4-Benzyl-naphthalin-1-ol,
- 4-Chlor-naphthalin-1-ol,
- 4-Chlor-5,8-dimethoxy-6-methyl-naphthalin-1-ol,
- 4-Chlor-5,8-dimethoxy-naphthalin-1-ol,
- 4-Acetoxy-5-chlor-6-methyl-7-acetyl-8-hydroxy-naphthalin-1-ol,
- 4-Acetoxy-6-methyl-7-acetyl-8-hydroxy-naphthalin-1-ol,
- 4-Acetoxy-8-benzyloxy-naphthalin-1-ol,
- 4-Benzyloxy-naphthalin-1-ol,
- 4,8-Dibenzyloxy-6-methyl-naphthalin-1-ol,
- 4,8-Dibenzyloxy-naphthalin-1-ol,
- 4-Benzyloxy-8-(2-chlor)ethoxy-naphthalin-1-ol,
- 4-Benzyloxy-8-isopropyloxy-naphthalin-1-ol,
- 4-Benzyloxy-8-methoxy-naphthalin-1-ol,
- 4-(2,2,2-Trifluorethoxy)-naphthalin-1-ol,
- 4-(2-Brom)ethoxy-naphthalin-1-ol,
- 4-(2-Brom)ethoxy-5-methoxy-naphthalin-1-ol,
- 4-(2-Brom)ethoxy-8-methoxy-naphthalin-1-ol,
- 4-(2-Chlor)ethoxy-naphthalin-1-ol,
- 4-(2-Chlor)ethoxy-8-methoxy-naphthalin-1-ol,
- 4-(2-Methoxy)ethoxy-naphthalin-1-ol,
- 4-(1,4,7-Trioxaheptyl)-naphthalin-1-ol,
- 4-(1,4,7-Trioxaoctyl)-naphthalin-1-ol,
- 4-(1,4,7,10-Tetraoxadecyl)-naphthalin-1-ol,
- (4-Hydroxy-1-naphthalinyl)-oxy-essigsäure,
- 4-Methoxy-naphthalin-1-ol,
- 4-Methoxy-5-chlor-naphthalin-1-ol,
- 4-Methoxy-5-chlor-8-benzyloxy-naphthalin-1-ol,
- 4,8-Dimethoxy-5-chlor-naphthalin-1-ol,
- 4-Methoxy-5-methyl-naphthalin-1-ol,
- 4-Methoxy-5-benzyloxy-naphthalin-1-ol,
- 4-Methoxy-5-benzyloxy-7-methyl-naphthalin-1-ol,
- 4-Methoxy-5-hydroxy-naphthalin-1-ol,
- 4-Methoxy-5-hydroxy-7-methyl-naphthalin-1-ol,
- 4-Methoxy-5-isopropyloxy-naphthalin-1-ol,
- 4,5-Dimethoxy-naphthalin-1-ol,
- 4,5-Dimethoxy-6-benzyloxy-naphthalin-1-ol,
- 4,5-Dimethoxy-7-methyl-naphthalin-1-ol,
- 4,5-Dimethoxy-8-chlor-naphthalin-1-ol,
- 4-Methoxy-6-methyl-naphthalin-1-ol,
- 4-Methoxy-6-methyl-7-acetyl-8-hydroxy-naphthalin-1-ol,
- 4-Methoxy-6,7-dimethyl-naphthalin-1-ol,
- 4-Methoxy-6-methyl-8-benzyloxy-naphthalin-1-ol,
- 4-Methoxy-6-methyl-8-hydroxy-naphthalin-1-ol,
- 4,8-Dimethoxy-6-methyl-naphthalin-1-ol,
- 4-Methoxy-6-ethoxy-naphthalin-1-ol,
- 4-Methoxy-6,7-diethoxy-naphthalin-1-ol,
- 4-Methoxy-7-methyl-naphthalin-1-ol,
- 4,8-Dimethoxy-7-benzyloxy-naphthalin-1-ol,
- 4-Methoxy-7-ethoxy-naphthalin-1-ol,
- 4-Methoxy-8-chlor-naphthalin-1-ol,
- 4-Methoxy-8-methyl-naphthalin-1-ol,
- 4-Methoxy-8-benzyloxy-naphthalin-1-ol,
- 4-Methoxy-8-hydroxy-naphthalin-1-ol,
- 4-Methoxy-8-isopropyloxy-naphthalin-1-ol,
- 4,8-Dimethoxy-naphthalin-1-ol,
- 4-Ethoxy-naphthalin-1-ol,
- 4-Propyloxy-naphthalin-1-ol,
- 4-Isopropyloxy-naphthalin-1-ol,
- 4-Butoxy-naphthalin-1-ol,
- 4-Isobutoxy-naphthalin-1-ol,
- 4-*sec*-butoxy-naphthalin-1-ol,
- 4-Isoamoxy-naphthalin-1-ol,
- 4-Bis(2-chlor-isopropyloxy)-naphthalin-1-ol,
- 4-Cyclohexyloxy-naphthalin-1-ol,
- 4-Octyloxy-naphthalin-1-ol,
- 4-(2-Chlor-propoxy)-naphthalin-1-ol,
- 4,5-Isopropyliden-dioxy-naphthalin-1-ol,
- 5-Methoxy-naphthalin-1-ol,
- 5,8-Dimethoxy-6-methyl-naphthalin-1-ol,
- 5,8-Dimethoxy-6,7-dichlor-naphthalin-1-ol,
- 5,8-Dimethoxy-7-methyl-naphthalin-1-ol,
- 5,8-Diacetoxy-naphthalin-1-ol und
- 8-Methoxy-naphthalin-1-ol.

12. Verfahren nach Anspruch 11, dadurch gekennzeichnet, daß die Verbindungen der Formel (I) ausgewählt sind unter:
- 4-Methoxy-naphthalin-1-ol,
- 4-Ethoxy-naphthalin-1-ol,
- 4-Isopropyloxy-naphthalin-1-ol und
- 4,8-Dimethoxy-naphthalin-1-ol.

13. Verfahren nach einem der vorhergehenden Ansprüche, dadurch gekennzeichnet, daß die Verbindung oder die Verbindungen der Formel (I) 0,001 bis 5 Gew.-%, bezogen auf das Gesamtgewicht der Zusammensetzung B1, ausmachen.

14. Verfahren nach einem der vorhergehenden Ansprüche, dadurch gekennzeichnet, daß der pH-Wert der Zusammensetzung A im Bereich von 3 bis 9 liegt.

15. Verfahren nach einem der vorhergehenden Ansprüche, dadurch gekennzeichnet, daß der pH-Wert der Zusammensetzung B größer oder gleich 6 ist.

16. Verfahren nach einem der vorhergehenden Ansprüche, dadurch gekennzeichnet, daß die Zusammensetzung B1 eine oder mehrere Oxidationsbasen und/oder einen oder mehrere Kuppler und/oder einen oder mehrere Direktfarbstoffe enthält.

17. Verfahren nach einem der vorhergehenden Ansprüche, dadurch gekennzeichnet, daß die in der Zusammensetzung B1 verwendbaren Additionssalze der Oxidationsbasen und/oder Kuppler mit einer Säure ausgewählt sind unter Hydrochloriden, Hydrobromiden, Sulfaten und Tartraten sowie Lactaten und Acetaten.

18. Verfahren nach einem der vorhergehenden Ansprüche, dadurch gekennzeichnet, daß das in der Zusammensetzung B2 vorliegende Oxidationsmittel ausgewählt ist unter Wasserstoffperoxid, Harnstoffperoxid, Iodaten und Periodaten, Metalloxiden, Metallchloriden, Ferrocyanid, Alkalimetallbromaten und Salzen von Persäuren, wie Perboraten und Persulfaten.

19. Verfahren nach Anspruch 18, dadurch gekennzeichnet, daß es sich bei dem Oxidationsmittel um Wasserstoffperoxid handelt.

20. Verfahren nach einem der vorhergehenden Ansprüche, dadurch gekennzeichnet, daß das zum Färben geeignete Medium (oder der Träger) aus Wasser oder einem Gemisch von Wasser und mindestens einem organischen Lösungsmittel, das unter niederen Alkanolen mit 1 bis 4 Kohlenstoffatomen, Glycerin, Glykolen und Glykolethern, aromatischen Alkoholen, analogen Produkten und deren Gemischen ausgewählt ist, besteht.

21. Vorrichtung mit mehreren Abteilung oder 'Kit' zum Färben mit mehreren Abteilungen, dadurch gekennzeichnet, daß eine erste Abteilung eine Zusammensetzung A, die in einem zum Färben geeigneten Medium mindestens ein Mangansalz und/oder mindestens einen Mangankomplex enthält, eine zweite Abteilung eine Zusammensetzung B1, die in einem zum Färben geeigneten Medium mindestens eine in einem der Ansprüche 1, 11 oder 12 definierte Verbindung der Formel (I) enthält, und eine dritte Abteilung eine Zusammensetzung B2 enthält, die in einem zum Färben geeigneten Medium mindestens ein Oxidationsmittel enthält, wobei der pH-Wert der Zusammensetzungen B1 und B2 so eingestellt ist, daß nach dem Mischen der Zusammensetzung B1 mit der Zusammensetzung B2 in einem Gewichtsverhältnis, das im Bereich von 0,5 bis 5 liegt, der pH-Wert der resultierenden Zusammensetzung B größer oder gleich 6 ist.
